Europäisches Patentamt

European Patent Office

Office européen des brevets

(11) Veröffentlichungsnummer: **0 509 268 A1**

# EUROPÄISCHE PATENTANMELDUNG

(21) Anmeldenummer: **92104865.8**

(22) Anmeldetag: **20.03.92**

(51) Int. Cl.5: **A61K 35/78**, A61K 31/085, A61K 31/015

(30) Priorität: **19.04.91 DE 4112824**

(43) Veröffentlichungstag der Anmeldung:
**21.10.92 Patentblatt 92/43**

(84) Benannte Vertragsstaaten:
**AT CH DE FR GB IT LI**

(71) Anmelder: **CHIMICASA GMBH**
**Wiesentalstrasse 81**
**CH-7000 Chur(CH)**

(72) Erfinder: **Deininger, Rolf, Dr.**
**Fürst-Pückler-Strasse 44**
**W-5000 Köln 41(DE)**

(74) Vertreter: **Hach, Hans Karl, Dr.**
**Tarunstrasse 23**
**W-6950 Mosbach-Waldstadt(DE)**

(54) **Verwendung von Nelkenöl zur Behandlung von BPH.**

(57) Verwendung von Nelkenöl zur Behandlung der Benignen Prostatahyperplasie.

EP 0 509 268 A1

Benigne Prostatahyperplasie (BPH) wird mit Extrakten aus Brennesselwurzel, Pappelrinde, Sägepalmfrüchten, Kürbissamen und weiblichen Keimdrüsenhormonen behandelt, bei den pflanzlichen Präparaten mit unbefriedigendem Erfolg, bei den Hormonpräparaten mit unerwünschten Nebenwirkungen. Da eine endgültige Heilung erfahrungsgemäß nicht erzielbar ist, muß oft auf operative Eingriffe ausgewichen werden.

Aufgabe der Erfindung ist es, ein verträgliches, zur wirksamen Behandlung der BPH geeignetes Präparat zur Verfügung zu stellen.

Diese Aufgabe wird gelöst durch die Verwendung von aus Gewürznelken (Flos Caryophylli; Syzygium aromaticum (L.) MERRIL et L. M. PERRY Synonym: Eugenia caryophyllata THUNBERG) gewonnenem ätherischen Öl - sogenanntem Nelkenöl - zur Behandlung der Benignen Prostatahyperplasie (BPH) durch orale oder anale Einnahme.

Versuche haben gezeigt, daß bei einer sich über 3 Monate erstreckenden Behandlung mit einer Tagesdosis von 3 x 100 mg Nelkenöl eine Verkleinerung der Prostata und im Gefolge davon eine hochsignifikante Verminderung des Restharns von anfangs im Mittel 89,8 ml auf 53,9 ml und eine erhebliche Erhöhung der maximalen Sekundenvolumina der Miktion erzielbar ist.

Aus der DE-OS 2719623 ist die Verwendung von Eugenol als Spasmolytikum bekannt. Eugenol ist einer der Hauptbestandteile des Nelkenöl und es ist anzunehmen, daß die erzielte Wirkung des Nelkenöls auf diesen Eugenol-Bestandteil zurückgeht. Die bekannte spasmolytische Wirkung steht aber in keinem Zusammenhang mit der hier verwendeten Wirkung des Nelkenöls und läßt diese auch nicht erwarten.

Das Präparat kann verabfolgt werden in Form von Gelatinekapseln, Dragees, Tabletten, Lösungen, Zäpfchen und dergleichen oral oder anal.

Nelkenöl aus Gewürznelken ist beschrieben und charakterisiert in einer Monografie im Deutschen Arzneibuch, 9. Ausgabe, 1986, Seite 835 und 836.

Es kann Nelkenöl eingesetzt werden, das durch Wasserdampfdestillation oder durch Extraktion mit unipolaren Lösungsmitteln, wie zum Beispiel Ethylalkohol, Methylalkohol oder Fregen, gewonnen ist aus ganzen oder zerkleinerten Blütenknospen, Blütenstielen und/oder Laubblättern von Gewürznelken.

Die Zusammensetzung des gewonnenen Nelkenöls ist als Naturprodukt abhängig vom Ausgangsmaterial. Bewährt hat sich Nelkenöl, das enthält 80 - 90 %, vorzugsweise 83 %, Eugenol, 10 - 15 %, vorzugsweise 11 % Aceteugenol und 5 - 12 %, vorzugsweise 6 %, alpha- und beta-Caryophyllen und Caryophyllenoxid.

Bei Behandlung der BPH im Stadium IV nach Vahlensieck, also bei permanenter Miktionsstörung, Miktion 10 ml/sec., Restharn 100 ml, Dilatationsblase, Harnstauung in den oberen Harnwegen, wurden gute Ergebnisse erzielt, indem über 1 bis 12 Monate, vorzugsweise 2 bis 5 Monate, Nelkenöl in einer Tagesdosis von 100 bis 1000 mg, vorzugsweise 250 bis 400 mg, zur Behandlung einer 70 kg schweren erwachsenen Person oral und zur Behandlung von Personen mit anderem Körpergewicht in einer proportional dem Körpergewicht angepaßten, geänderten Tagesdosis verabfolgt wurde und indem über 1 bis 12 Monate, vorzugsweise 2 bis 5 Monate, Nelkenöl in einer Tagesdosis von 200 bis 2000 mg, vorzugsweise 400 bis 800 mg, zur Behandlung einer 70 kg schweren erwachsenen Person anal und zur Behandlung von Personen mit anderem Körpergewicht in einer proportional dem Körpergewicht angepaßten, geänderten Tagesdosis verabfolgt wurde.

Die Erfindung wird nun anhand einiger Versuchsbeispiele näher erläutert.

BEISPIEL 1

Es wurde eine klinische Prüfung wie folgt durchgeführt:

25 Patienten wurde 3 x täglich eine Kapsel nach den Mahlzeiten oral verabfolgt. Jede Kapsel enthielt 200 mg Nelkenöl. Dieses Nelkenöl hatte eine Zusammensetzung wie folgt:

23 % Eugenol

11 % Aceteugenol

6 % alpha und beta Caryophyllen und Caryophyllenoxid.

Die Ausgangssituation für die klinische Prüfung war wie folgt:

```
Zahl der rekrutierten Patienten:        25

Altersdurchschnitt (Jahre):             69,5    (57-85)

Durchschnittliche Dauer der
Miktionsstörungen (Monate):             31,5    (1-140)

Durchschnittliche Miktionsfrequenz
tags:                                   7,7 x  (3-20)
nachts:                                 2,8 x  (0-6)

Imperativer Harndrang      nie:         5 Patienten
                      zeitweise:       16 Patienten
                          immer:        4 Patienten

Harnröhrenschmerz (7 Patienten)
                 vor Miktion:           4 Patienten
              während Miktion:          2 Patienten
                 nach Miktion:          4 Patienten

durchschnittliche Uroflow-Ergebnisse
          Max. Sek. Vol. (ml):         15   (3-36)
              Miktionszeit (s):        46,3 (12-180)
           Miktionsvolumen (ml):      245   (100-480)

durchschnittliche Restharnmenge
(ml, sonographisch oder radio-
logisch):                              96   (0-335)

Restharnfreie Miktion                   1 Patient
```

durchschnittliche Prostatagröße
(palpatorisch, radiologisch,
sonographisch)          normal:          4 Patienten
                schwach vergrößert:     12 Patienten
                  stark vergrößert:      9 Patienten

Prostatakongestion:                      3 Patienten

positiver Uricult-Befund:              _ 4 Patienten

Die im Anschluß an die 3monatige Behandlung durchgeführte
Kontrolluntersuchung ergab folgendes Ergebnis:

Durchschnittliche Behandlungs-/
Beobachtungsdauer (Monate):              3,0    (1-4)

Besserung der Miktion:                  21 Patienten

Keine Besserung der Miktion:             4 Patienten

Durchschnittliche Miktionsfrequenz
                        tags:           6,2 x (3-12)
                      nachts:           1,3 x (0-5)

Harnröhrenschmerz (3 Patienten)
                 vor Miktion:           1 Patient
             während Miktion:           1 Patient
                nach Miktion:           2 Patienten

Durchschnittliche Uroflow-Ergebnisse
        Max. Sek. Vol. (ml):           19,7  (5-29)
             Miktionszeit (s):          36,6  (23-60)
        Miktionsvolumen (ml):         312,9  (160-840)

Durchschnittliche Restharnmenge
(ml, sonographisch oder radiologisch):                               58,7  (0-320)

Restharnfreie Miktion:                   3 Patienten

Verkleinerung der Prostata
                palpatorisch:           Ø
                radiologisch:           Ø
              sonographisch:            3 Patienten
                        (von 150g verringert auf 130g,
                         von  45g verringert auf  26g,
                         von  45g verringert auf  36g)

Urteil der Prüfärzte   Besserung:      21 Patienten
                      Status idem:      3 Patienten
                 Verschlechterung:      1 Patient

4

Nebenwirkungen wurden bei 5 Patienten beobachtet, und zwar leichte Übelkeit 1/2 Stunde nach Tabletteneinnahme, Aufstoßen 1 Stunde nach Tabletteneinnahme, Kopfschmerzen in den ersten 3 Tagen der Therapie und vorübergehende "Magenstörungen".

In zwei Fällen mußte die Therapie abgebrochen werden wegen starker Zunahme der Nykturie 5 Wochen nach Therapiebeginn und Makrohämaturie mit Harnverhalt.

## Patentansprüche

1. Verwendung von aus Gewürznelken (Flos Caryophylli; Syzygium aromaticum (L.) MERRIL et L. M. PERRY Synonym: Eugenia caryophyllata THUNBERG) gewonnenem ätherischen Öl - sogenanntem Nelkenöl - zur Behandlung der Benignen Prostatahyperplasie (BPH) durch orale oder anale Einnahme.

2. Verwendung nach Anspruch 1, dadurch gekennzeichnet, daß Nelkenöl eingesetzt wird, das durch Wasserdampfdestillation gewonnen ist aus ganzen oder zerkleinerten Blütenknospen, Blütenstielen und/oder Laubblättern von Gewürznelken.

3. Verwendung nach Anspruch 1, dadurch gekennzeichnet, daß Nelkenöl eingesetzt wird, das durch Extraktion mit unipolaren Lösungsmitteln gewonnen ist aus den ganzen oder zerkleinerten Blütenknospen, Blütenstielen und/oder Laubblättern von Gewürznelken.

4. Verwendung nach einem der vorhergehenden Ansprüche, dadurch gekennzeichnet,
   daß Nelkenöl eingesetzt wird, das enthält:
   80 - 90 %, vorzugsweise 83 %, Eugenol,
   10 - 15 %, vorzugsweise 11 %, Aceteugenol
   5 - 12 %, vorzugsweise 6 %, alpha- und beta-Caryophyllen und Caryophyllenoxid.

5. Verwendung nach einem der vorhergehenden Ansprüche, dadurch gekennzeichnet,
   daß über 1 bis 12 Monate, vorzugsweise 2 bis 5 Monate, Nelkenöl in einer Tagesdosis von 100 bis 1000 mg, vorzugsweise 250 bis 400 mg, zur Behandlung einer 70 kg schweren erwachsenen Person oral und zur Behandlung von Personen mit anderem Körpergewicht in einer proportional dem Körpergewicht angepaßten, geänderten Tagesdosis verabfolgt wird.

6. Verwendung nach einem der Ansprüche 1 bis 4, dadurch gekennzeichnet,
   daß über 1 bis 12 Monate, vorzugsweise 2 bis 5 Monate, Nelkenöl in einer Tagesdosis von 200 bis 2000 mg, vorzugsweise 400 bis 800 mg, zur Behandlung einer 70 kg schweren erwachsenen Person anal und zur Behandlung von Personen mit anderem Körpergewicht in einer proportional dem Körpergewicht angepaßten, geänderten Tagesdosis verabfolgt wird.

Europäisches
Patentamt

**EUROPÄISCHER TEILRECHERCHENBERICHT**

der nach Regel 45 des Europäischen Patentübereinkommens für das weitere Verfahren als
europäischer Recherchenbericht gilt

Nummer der Anmeldung

EP  92 10 4865

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl.5) |
|---|---|---|---|
| A,P | ZEITSCHRIFT FÜR PHYTOTHERAPIE, Band 12, Nr. 6, 15. Dezember 1991, Seiten 205-212, Hippokrates Verlag GmbH, Stuttgart, DE; R. DEININGER: "Gewürznelken (Syzygium aromaticum) und Nelkenöl – aktuelle Phytopharmaka" * Das ganze Artikel * ----- | 1 | A 61 K  35/78<br>A 61 K  31/085<br>A 61 K  31/015 |

**RECHERCHIERTE SACHGEBIETE (Int. Cl.5)**

A 61 K

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich
ist, auf der Grundlage einiger Patentansprüche sinnvolle Ermittlungen über den Stand der
Technik durchzuführen.
Vollständig recherchierte Patentansprüche:
Unvollständig recherchierte Patentansprüche:
Nicht recherchierte Patentansprüche:
Grund für die Beschränkung der Recherche:

Bemerkung:Obwohl die Ansprüche 1 bis 6 sich
auf ein Verfahren zur Behandlung des mensch-
lichen/tierischen Körpers beziehen (Art. 52(4)
EPÜ), wurde die Recherche durchgeführt und
gründete sich auf die angeführten Wirkungen
der Verbindung/Zusammensetzung

| Recherchenort | Abschlußdatum der Recherche | Prüfer |
|---|---|---|
| DEN HAAG | 16-07-1992 | REMPP G.L.E. |

EPO FORM 1503 03.82 (P0409)